# EUROPÄISCHE PATENTSCHRIFT

(11) **EP 0 952 190 B1**
(45) Veröffentlichungstag und Bekanntmachung des Hinweises auf die Patenterteilung: **04.06.2003**
(21) Anmeldenummer: 99810309.7
(22) Anmeldetag: 14.04.1999
(51) Int. Cl.: C09B 29/085, C07C 233/43, D06P 3/54, B41M 5/38

(54) **Dispersionsfarbstoffe**
Dispersion dyestuffs
Colorants de dispersion

(30) Priorität: 23.04.1998 EP 98810359
(43) Veröffentlichungstag der Anmeldung: 27.10.1999
(73) Patentinhaber: Ciba Specialty Chemicals Holding Inc., 4057 Basel (CH)
(72) Erfinder: Clément, Antoine, 4058 Basel (CH); Arquint, Alfons, 4058 Basel (CH); Lauk, Urs, 8047 Zürich (CH)

(56) Entgegenhaltungen:
- EP-A- 0 555 179
- EP-A- 0 623 654
- EP-A- 0 826 741
- US-A- 3 406 165

## Beschreibung

Gegenstand der vorliegenden Erfindung sind Dispersionsfarbstoffe, Verfahren zu deren Herstellung sowie ihre Verwendung zum Färben oder Bedrucken von halbsynthetischen oder synthetischen hydrophoben Fasermaterialien.

Dispersionsfarbstoffe, d.h. Farbstoffe, welche keine wasserlöslichmachenden Gruppen enthalten, sind seit langem bekannt und werden zum Färben von hydrophoben Textilmaterialien verwendet. Vielfach sind die erhaltenen Färbungen jedoch nicht ausreichend thermomigrierecht und haben auch zum Teil unbefriedigende Echtheiten, insbesondere Wasch- und Schweissechtheiten. Dieses Problem tritt vor allem bei roten bis blauen Nuancen auf.

Gegenstand der vorliegenden Erfindung sind nun Dispersionsfarbstoffe, welche sehr thermomigrierechte und wasch- und schweissechte Färbungen ergeben, und zu dem sowohl im Auszieh- und Thermosolverfahren als auch im Textildruck ein gutes Aufbauvermögen besitzen. Die Farbstoffe sind auch für den Ätzdruck geeignet.

Die erfindungsgemässen Farbstoffe entsprechen der Formel 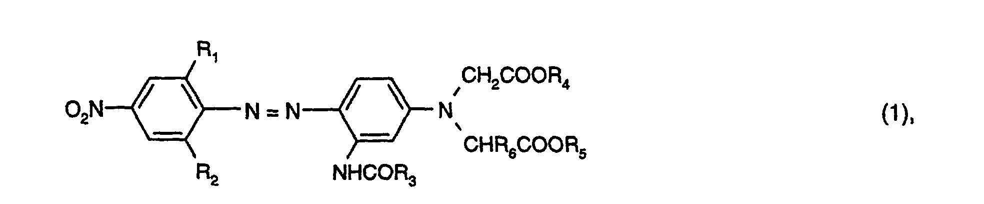
worin R₁ Nitro oder Cyano, R₂ Halogen, R₃ C₁-C₄-Alkyl, R₄ und R₅ unabhängig voneinander Methyl oder Ethyl und R₆ Methyl oder Ethyl sind.
R₂ als Halogen ist z.B. Chlor, Brom, oder Jod.
R₃ als C₁-C₄-Alkyl ist z.B. Methyl, Ethyl, n-Propyl, Isopropyl, n-Butyl, sec.-Butyl, tert.-Butyl oder Isobutyl.
R₂ ist bevorzugt Chlor oder Brom und insbesondere Brom.
R₃ ist bevorzugt C₁-C₃-Alkyl, insbesondere Methyl, Ethyl oder Isopropyl. Besonders bevorzugt ist R₃ Methyl oder Ethyl und insbesondere Methyl.
R₆ ist bevorzugt Methyl.

Bevorzugt sind Farbstoffe der Formel (1), worin
R₁ Cyano, R₂ Chlor oder Brom, R₃ C₁-C₃-Alkyl, insbesondere Methyl oder Ethyl und ganz besonders Methyl, R₄ und R₅ unabhängig voneinander Methyl oder Ethyl, insbesondere Methyl, und R₆ Methyl sind.

Aus EP, A, 555 179 sind Farbstoffe der Formel (1) bekannt, worin R₁ Nitro oder Cyano, R₂ Halogen, R₃ C₁-C₄-Alkyl, R₄ und R₅ Methyl und R₆ Wasserstoff sind.

Die Farbstoffe der Formel (1) können nach an sich bekannten Verfahren hergestellt werden. Man erhält sie beispielsweise, indem man eine Verbindung der Formel 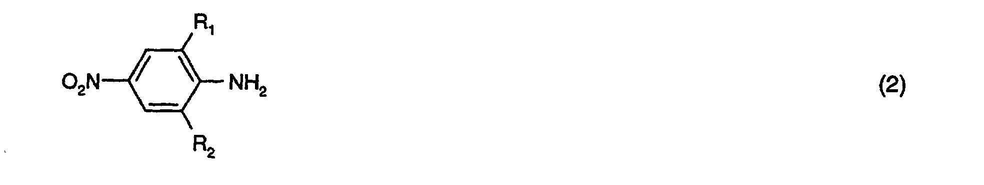 diazotiert und auf eine Kupplungskomponente der Formel 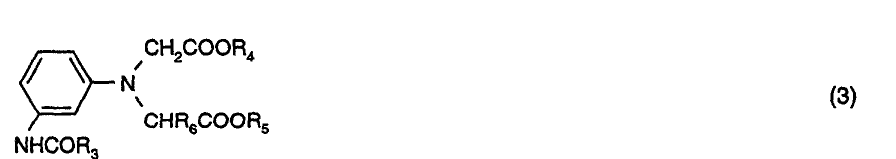 kuppelt, wobei R₁, R₂, R₃, R₄, R₅ und R₆ die unter der Formel (1) angegebenen Bedeutungen aufweisen.

Die Diazotierung der Verbindungen der Formel (2) erfolgt in an sich bekannter Weise, z.B. mit Natriumnitrit in saurem, z.B. salzsaurem oder schwefelsaurem, wässrigem Medium. Die Diazotierung kann aber auch mit anderen Diazotierungsmitteln, z.B. mit Nitrosylschwefelsäure ausgeführt werden. Bei der Diazotierung kann eine zusätzliche Säure im Reaktionsmedium anwesend sein, z.B. Phosphorsäure, Schwefelsäure, Essigsäure, Propionsäure, Salzsäure oder Mischungen dieser Säuren, z.B. Mischungen aus Phosphorsäure und Essigsäure. Zweckmässig wird die Diazotierung bei Temperaturen von -10 bis 30°C, vorzugsweise von -10°C bis Raumtemperatur, durchgeführt.

Die Kupplung der diazotierten Verbindung der Formel (2) auf die Kupplungskomponente der Formel (3) erfolgt ebenfalls in bekannter Weise, beispielsweise in saurem, wässrigem oder wässrig-organischem Medium, vorteilhaft bei Temperaturen von -10 bis 30°C, insbesondere unter 10°C. Als Säuren verwendet man z.B. Salzsäure, Essigsäure, Schwefelsäure oder Phosphorsäure. Diazotierung und Kupplung können beispielsweise im gleichen Reaktionsmedium durchgeführt werden.

Die Diazokomponenten der Formel (2) und die Kupplungskomponenten der Formel (3) sind z. T. bekannt oder können auf an sich bekannte Art und Weise hergestellt werden.

Die Kupplungskomponente der Formel 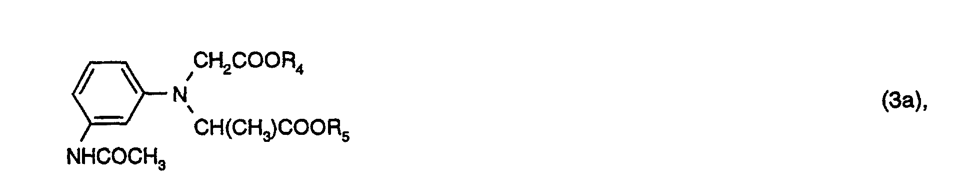 worin R₄ und R₅ die unter der Formel (1) angegebene Bedeutung haben, ist neu und stellt einen weiteren Gegenstand der vorliegenden Erfindung dar.

Die Kupplungskomponente der Formel (3a) wird z.B. hergestellt, indem man 3-Amino-acetanilid zuerst mit einer Verbindung der Formel CH₃-CHCl-COOR₅ und anschliessend mit einer Verbindung der Formel Cl-CH₂-COOR₄ umsetzt.

Die erfindungsgemässen Farbstoffe der Formel (1) können zum Färben und Bedrucken von halbsynthetischen und insbesondere synthetischen hydrophoben Fasermaterialien, vor allem Textilmaterialien, verwendet werden. Textilmaterialien aus Mischgeweben, die derartige halbsynthetische bzw. synthetische hydrophobe Fasermaterialien enthalten, können ebenfalls mit Hilfe der erfindungsgemässen Farbstoffe gefärbt oder bedruckt werden.

Als halbsynthetische Textilmaterialien kommen vor allem Cellulose-2½-Acetat und Cellulosetriacetat in Frage.

Synthetische hydrophobe Textilmaterialien bestehen vor allem aus linearen, aromatischen Polyestern, beispielsweise solchen aus Terephthalsäure und Glykolen, besonders Ethylenglykol oder Kondensationsprodukten aus Terephthalsäure und 1,4-Bis-(hydroxymethyl)-cyclohexan; aus Polycarbonaten, z.B. solchen aus α,α-Dimethyl-4,4'-dihydroxy-diphenylmethan und Phosgen, aus Fasern auf Polyvinylchlorid- sowie Polyamid-Basis.

Die Applikation der erfindungsgemässen Farbstoffe auf die Textilmaterialien erfolgt nach bekannten Färbeverfahren. Beispielsweise färbt man Polyesterfasermaterialien im Ausziehverfahren aus wässriger Dispersion in Gegenwart von üblichen anionischen oder nichtionischen Dispergiermitteln und gegebenenfalls üblichen Quellmitteln (Carrier) bei Temperaturen zwischen 80 und 140°C. Cellulose-2½-acetat färbt man vorzugsweise zwischen ungefähr 65 bis 85°C und Cellulosetriacetat bei Temperaturen bis zu 115°C.

Die erfindungsgemässen Farbstoffe färben im Färbebad gleichzeitig anwesende Wolle und Baumwolle nicht oder nur wenig an (sehr gute Reserve), so dass sie auch gut zum Färben von Polyester/Wolle- und Polyester/Cellulosefaser-Mischgeweben verwendet werden können.

Die erfindungsgemässen Farbstoffe eignen sich zum Färben nach dem Thermosolverfahren, im Auszieh- und Continueverfahren und für Druckverfahren. Das Ausziehverfahren ist bevorzugt. Das Flottenverhältnis ist von den apparativen Gegebenheiten, vom Substrat und der Aufmachungsform abhängig. Es kann jedoch innerhalb eines weiten Bereiches gewählt werden, z.B. 1:4 bis 1:100, liegt aber vorzugsweise zwischen 1:6 bis 1:25.

Das genannte Textilmaterial kann dabei in den verschiedenen Verarbeitungsformen vorliegen, wie z.B. als Faser, Faden oder Vlies, als Gewebe oder Gewirke.

Es ist vorteilhaft, die erfindungsgemässen Farbstoffe vor ihrer Verwendung in ein Farbstoffpräparat zu überführen. Hierzu wird der Farbstoff vermahlen, so dass seine Teilchengrösse im Mittel zwischen 0,1 und 10 Mikron beträgt. Das Vermahlen kann in Gegenwart von Dispergiermitteln erfolgen. Beispielsweise wird der getrocknete Farbstoff mit einem Dispergiermittel gemahlen oder in Pastenform mit einem Dispergiermittel geknetet und hierauf im Vakuum oder durch Zerstäuben getrocknet. Mit den so erhaltenen Präparaten kann man nach Zugabe von Wasser Druckpasten und Färbebäder herstellen.

Beim Bedrucken wird man die üblichen Verdickungsmittel verwenden, z.B. modifizierte oder nichtmodifizierte natürliche Produkte, beispielsweise Alginate, British-Gummi, Gummi arabicum, Kristallgummi, Johannisbrotkernmehl, Tragant, Carboxymethylcellulose, Hydroxyethylcellulose. Stärke oder synthetische Produkte, beispielsweise Polyacrylamide, Polyacrylsäure oder deren Copolymere, oder Polyvinylalkohole.

Die erfindungsgemässen Farbstoffe sind beständig gegen Eisen- und Kupferionen und verleihen den genannten Materialien, vor allem dem Polyestermaterial, egale Farbtöne von sehr guten Gebrauchsechtheiten, wie guter Lichtechtheit und guter Sublimierechtheit. Hervorzuheben ist die sehr gute Wasch- und Schweissechtheit und vor allem die gute Thermomigrationsechtheit der erhaltenen Färbungen. Die erfindungsgemässen Farbstoffe zeichnen sich ferner durch einen guten Auszug und Aufbau aus.

Die erfindungsgemässen Farbstoffe können auch gut verwendet werden zur Herstellung von Mischnuancen sowohl untereinander als auch zusammen mit anderen Farbstoffen.

Die vorstehend genannten Verwendungen der erfindungsgemässen Farbstoffe stellt ebenso einen Gegenstand der vorliegenden Erfindung dar, wie ein Verfahren zum Färben oder Bedrucken von halbsynthetischem oder synthetischem hydrophobem Fasermaterial, insbesondere Textilmaterial, das darin besteht einen oder mehrere erfindungsgemässen Farbstoffe auf das genannte Material aufzubringen oder es in dieses einzuarbeiten. Das genannte hydrophobe Fasermaterial ist vorzugsweise textiles Polyestermaterial. Weitere Substrate, die durch das erfindungsgemässe Verfahren behandelt werden können sowie bevorzugte Verfahrensbedingungen sind vorstehend bei der näheren Erläuterung der Verwendung der erfindungsgemässen Farbstoffe zu finden.

Die erfindungsgemässen Farbstoffe der Formel (1) eignen sich ausserdem für moderne Aufzeichnungsverfahren, wie z.B. Thermotransfer-Printing.

Die nachfolgenden Beispiele dienen der Veranschaulichung der Erfindung. Darin sind, sofern nicht anders angegeben, die Teile Gewichtsteile und die Prozente Gewichtsprozente. Die Temperaturen sind in Celsiusgraden angegeben. Die Beziehung zwischen Gewichtsteilen und Volumenteilen ist dieselbe wie zwischen Gramm und Kubikzentimeter.

### Beispiel 1:

50,0 Teile 3-Amino-acetanilid werden bei einer Temperatur von 20 bis 30°C in 200 Teile 2-Chlor-propionsäure-methylester in einem Reaktionskolben eingetragen. Danach werden 66 Teile Natriumcarbonat zugegeben. Die entstandene Suspension wird gleichmässig unter ständigem Rühren auf 127°C aufgeheizt und 5 Stunden bei dieser Temperatur gehalten. Anschliessend wird der überschüssige 2-Chlor-propionsäure-methylester bei 125°C im Vakuum abdestilliert. Zur Reaktionsmischung werden dann 120 Teile Chloressigsäure-methylester zugegeben und die Mischung 8 Stunden bei 115°C gehalten. Danach wird die ausreagierte Mischung auf Raumtemperatur abgekühlt, mit 330 Teilen Wasser versetzt und 30 Minuten verrührt bis die Salze vollständig gelöst sind. Nach anschliessendem kurzen Stehenlassen bilden sich in dem Reaktionskolben 2 Phasen. Die untere, organische Phase wird abgetrennt. Am Rotationsverdampfer wird aus der organischen Phase der überschüssige Chloressigsäure-methylester abdestilliert. Die erhaltenen 105 Teile der Verbindung der Formel 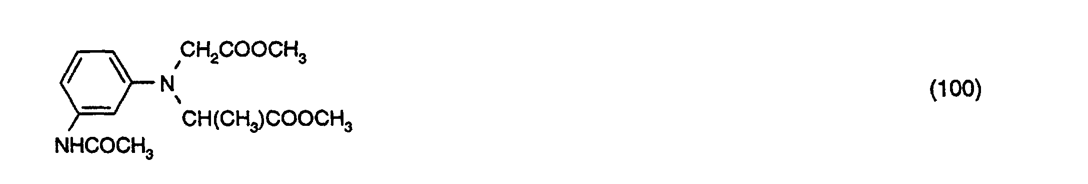 in Form eines harzigen Rückstandes werden in 195 Teilen Essigsäure gelöst.

### Beispiel 2:

In einem Reaktionskolben werden 78,6 Teile 2-Brom-4,6-dinitroanilin in 107 Teilen 98%-iger Schwefelsäure bei einer maximalen Temperatur von 35°C gelöst. Danach werden bei einer Temperatur zwischen 25 und 28°C 104 Teile 40%-iger Nitrosylschwefelsäure innerhalb 40 Minuten zum Reaktionsgemisch zugetropft und 120 Minuten bei 25°C nachgerührt. Die entstandene Diazolösung wird anschliessend innerhalb 60 Minuten bei 0 bis 5°C zu 300 Teilen der 35%-igen Lösung der Kupplungskomponente aus Beispiel 1 und 200 Teilen Eis zugetropft, wobei die Reaktionstemperatur durch Zugabe von Eis bei maximal 5° C gehalten wird. Nach der beendeten Zugabe der Diazolösung wird das Gemisch 2 Stunden nachgerührt, wobei die Temperatur auf 20°C ansteigt. Der entstandene Niederschlag wird abfiltriert und mit Wasser gewaschen. Nach dem Trocknen erhält man 153 Teile eines Farbstoffes der Formel 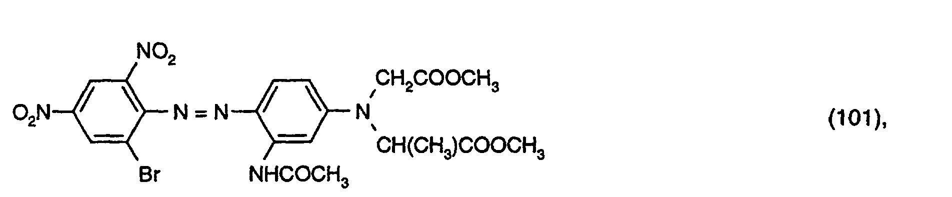 mit einem Absorptionsmaximum λₘₐₓ bei 516 nm, der Textilmaterial aus Polyester in einem rot-violetten Farbton mit guten Echtheiten färbt.

### Beispiele 3 - 17:

Analog der in den Beispielen 1 und 2 angegebenen Vorschriften lassen sich die in der Tabelle 1 aufgeführten Farbstoffe der Formeln (102) bis (116) herstellen, wenn man bei der Herstellung der Kupplungskomponente gemäss Beispiel 1 anstatt 50,0 Teilen 3-Aminoacetanilid eine äquimolare Menge der Verbindung der Formel 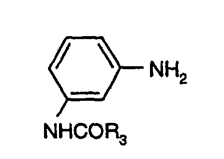 zunächst mit einer Verbindung der Formel CH₃-CHCl-COOR₅ und anschliessend mit einer Verbindung der Formel Cl-CH₂-COOR₄ umsetz, wobei R₄ und R₅ identisch oder verschieden sind, und bei der Diazotierung und Kupplung gemäss Beispiel 2 anstatt 78,6 Teile 2-Brom-4,6-dinitroanilin die äquimolare Menge der Verbindung der Formel 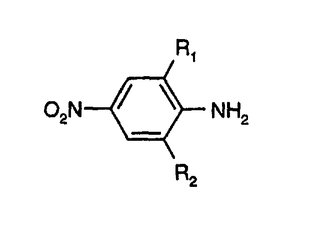 verwendet, wobei die Reste R₁, R₂, R₃, R₄, und R₅ die in Tabelle 1 für die jeweiligen Farbstoffe aufgeführten Bedeutungen haben.

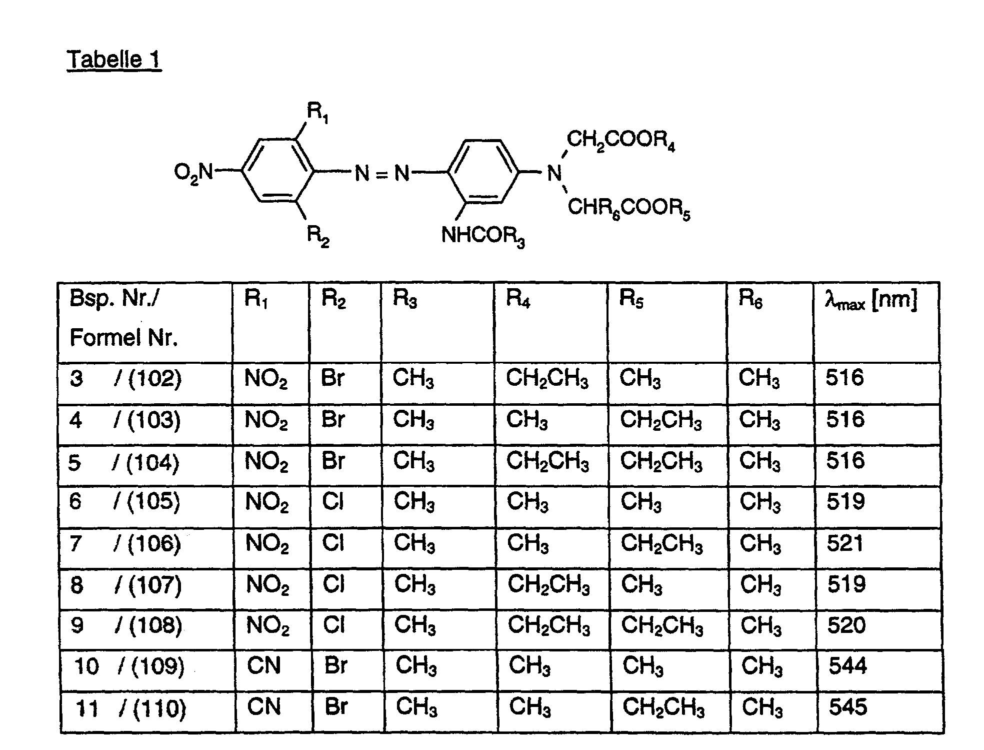

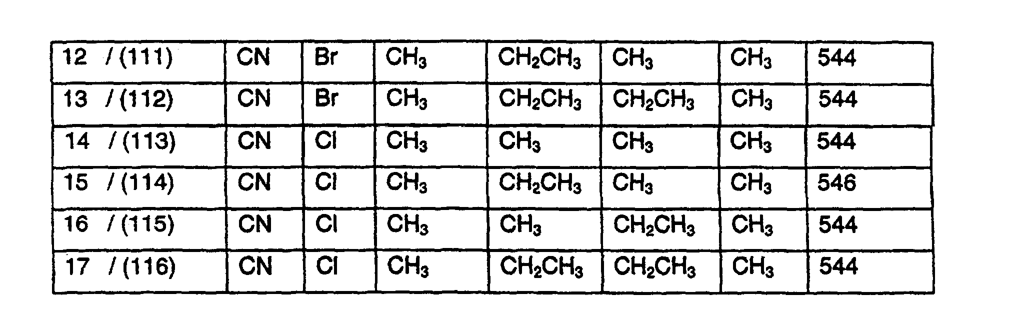

Die in Tabelle 1 aufgeführten Farbstoffe färben ebenfalls das Textilmaterial aus Polyester in violetten Farbtönen mit guten Echtheiten.

## Patentansprüche

1. Farbstoffe der Formel 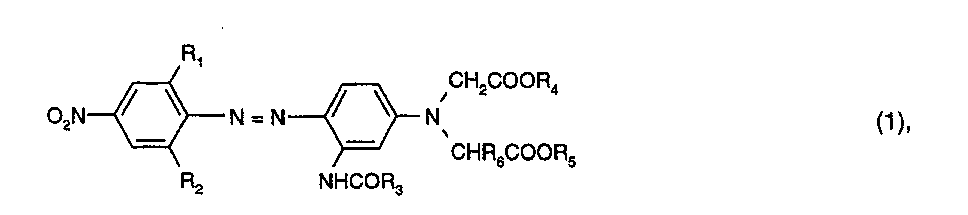 worin R₁ Nitro oder Cyano, R₂ Halogen, R₃ C₁-C₄-Alkyl, R₄ und R₅ unabhängig voneinander Methyl oder Ethyl und R₆ Methyl oder Ethyl sind.

2. Farbstoffe gemäss Anspruch 1, worin R₂ Chlor oder Brom ist.

3. Farbstoffe gemäss Anspruch 1 oder 2, worin R₂ Brom ist.

4. Farbstoffe gemäss einem der Ansprüche 1 bis 3, worin R₃ C₁-C₃-Alkyl und vorzugsweise Methyl ist.

5. Farbstoffe gemäss einem der Ansprüche 1 bis 4, worin R₆ Methyl ist.

6. Farbstoffe gemäss einem der Ansprüche 1 bis 5, worin R₁ Cyano, R₂ Chlor oder Brom, R₃ C₁-C₃-Alkyl, R₄ und R₅ unabhängig voneinander Methyl oder Ethyl und R₆ Methyl sind.

7. Farbstoffe gemäss einem der Ansprüche 1 bis 6, worin R₁ Cyano, R₂ Chlor oder Brom und R₃, R₄, R₅ und R₆ Methyl sind.

8. Verfahren zur Herstellung von Farbstoffen der Formel (1) gemäss Anspruch 1, **dadurch gekennzeichnet, dass** man eine Verbindung der Formel 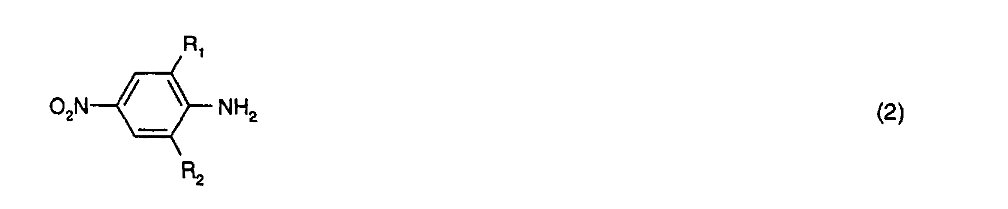 diazotiert und auf eine Kupplungskomponente der Formel 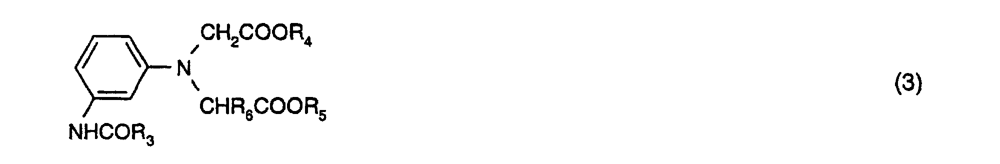 kuppelt, wobei R₁, R₂, R₃, R₄, R₅ und R₆ die unter der Formel (1) angegebenen Bedeutungen aufweisen.

9. Verfahren zum Färben oder Bedrucken von halbsynthetischem oder synthetischem hydrophobem Fasermaterial, insbesondere Textilmaterial, **dadurch gekennzeichnet, dass** man einen oder mehrere Farbstoffe gemäss Anspruch 1 auf das genannte Material aufbringt oder diesem einverleibt.

10. Verfahren nach Anspruch 9, worin das hydrophobe Material, vorzugsweise Textilmaterial, aus Polyesterfasern besteht.

11. Kupplungskomponente der Formel 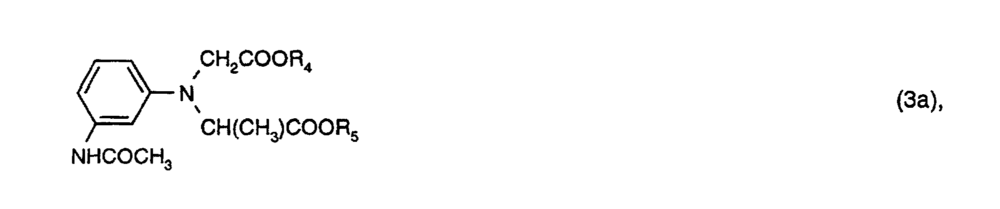 worin R₄ und R₅ unabhängig voneinander Methyl oder Ethyl sind.

12. Verfahren zur Herstellung der Kupplungskomponente der Formel (3a) gemäss Anspruch 11, **dadurch gekennzeichnet, dass** man 3-Amino-acetanilid zuerst mit einer Verbindung der Formel CH₃-CHCl-COOR₅ und anschliessend mit einer Verbindung der Formel Cl-CH₂-COOR₄ umsetzt, wobei R₄ und R₅ die unter der Formel (3a) angegebene Bedeutung haben.

## Claims

1. A dye of formula 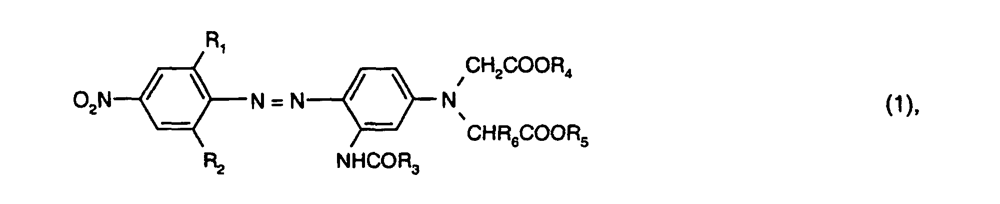 wherein R₁ is nitro or cyano, R₂ is halogen, R₃ is C₁-C₄alkyl, R₄ and R₅ are each independently of the other methyl or ethyl and R₆ is methyl or ethyl.

2. A dye according to claim 1, wherein R₂ is chloro or bromo.

3. A dye according to either claim 1 or 2, wherein R₂ is bromo.

4. A dye according to any one of claims 1 to 3, wherein R₃ is C₁-C₃alkyl and, preferably, methyl.

5. A dye according to any one of claims 1 to 4, wherein R₆ is methyl.

6. A dye according to any one of claims 1 to 5, wherein R₁ is cyano, R₂ is chloro or bromo, R₃ is C₁-C₃alkyl, R₄ and R₅ are each independently of the other methyl or ethyl and R₆ is methyl.

7. A dye according to any one of claims 1 to 6, wherein R₁ is cyano, R₂ is chloro or bromo and R₃, R₄, R₅ and R₆ are methyl.

8. A process for the preparation of a dye of formula (1) according to claim 1, which comprises diazotising a compound of formula 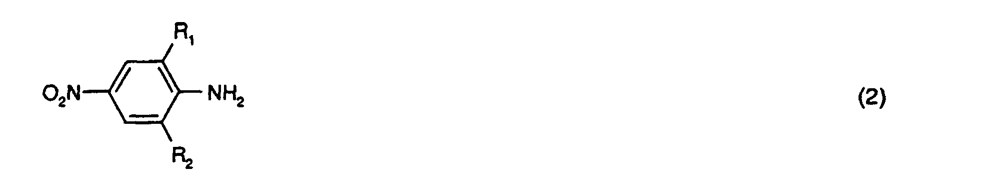 and coupling the diazonium compound to a coupling component of formula 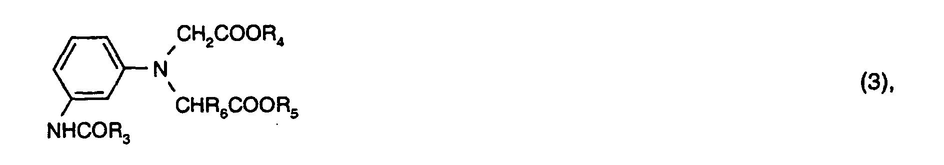 where R₁, R₂, R₃, R₄, R₅ and R₆ have the meanings cited under formula (1).

9. A process for dyeing or printing semisynthetic or synthetic hydrophobic fibre material, in particular textile material, which comprises applying to, or incorporating into, the cited material one or more than one dye according to claim 1.

10. A process according to claim 9, wherein the hydrophobic material, preferably textile material, consists of polyester fibres.

11. A coupling component of formula 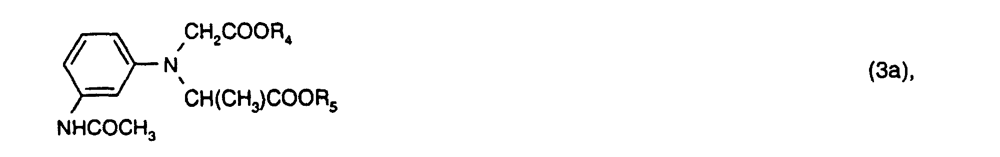 wherein R₄ and R₅ are each independently of the other methyl or ethyl.

12. A process for the preparation of the coupling component of formula (3a) according to claim 11, which comprises reacting 3-aminoacetanilide first with a compound of formula CH₃-CHCl-COOR₅ and then with a compound of formula Cl-CH₂-COOR₄, R₄ and R₅ having the meanings cited under formula (3a).

## Revendications

1. Colorants de formule 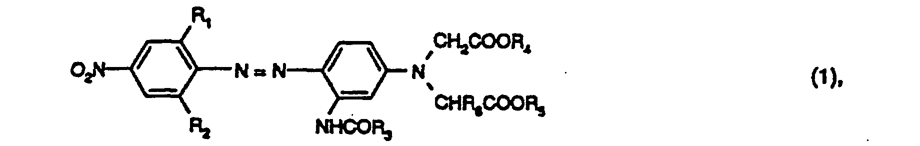 où R₁ représente un groupe nitro ou cyano, R₂ représente un atome d'halogène, R₃ représente un groupe alkyle en C₁-C₄, R₄ et R₅ représentent indépendamment l'un de l'autre un groupe méthyle ou éthyle et R₆ représente un groupe méthyle ou éthyle.

2. Colorants selon la revendication 1, où R₂ représente un atome de chlore ou de brome.

3. Colorants selon la revendication 1 ou 2, où R₂ représente un atome de brome.

4. Colorants selon l'une des revendications 1 à 3, où R₃ représente un groupe alkyle en C₁-C₃ et de préférence méthyle.

5. Colorants selon l'une des revendications 1 à 4, où R₆ représente un groupe méthyle.

6. Colorants selon l'une des revendications 1 à 5, R₁ représente un groupe cyano, R₂ représente un atome de chlore ou de brome, R₃ représente un groupe alkyle en C₁-C₃, R₄ et R₅ représentent indépendamment l'un de l'autre, un groupe méthyle ou éthyle et R₆ représente un groupe méthyle.

7. Colorants selon l'une des revendications 1 à 6, R₁ représente un groupe cyano, R₂ représente un atome de chlore ou de brome et R₃, R₄, R₅ et R₆ représentent un groupe méthyle.

8. Procédé pour la préparation de colorants de formule (1) selon la revendication 1, **caractérisé en ce qu'**on diazote un composé de formule 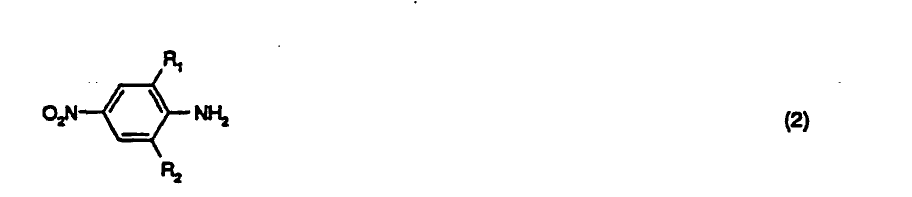 et on le copule sur un composant de copulation de formule 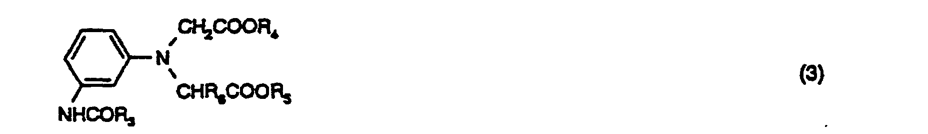 R₁, R₂, R₃, R₄, R₅ et R₆ possèdent les significations données à la formule (1).

9. Procédé pour la teinture ou l'impression de matières fibreuses hydrophobes semi-synthétiques ou synthétiques, en particulier de matière textile, **caractérisé en ce qu'**on applique un ou plusieurs colorants selon la revendication 1 à la matière précitée ou on l'incorpore à celle-ci.

10. Procédé selon la revendication 9, où la matière hydrophobe, de préférence la matière textile, est constituée par des fibres de polyester.

11. Composant de copulation de formule 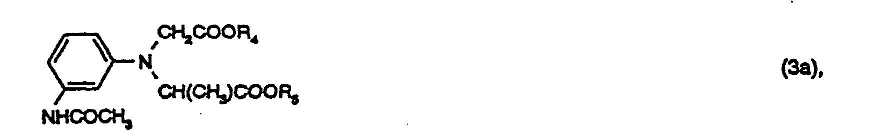 où R₄ et R₅ représentent indépendamment l'un de l'autre un groupe méthyle ou éthyle.

12. Procédé pour la préparation du composant de copulation de formule (3a) selon la revendication 11, **caractérisé en ce qu'**on fait réagir d'abord le 3-amino-acétanilide sur un composé de formule CH₃-CHCl-COOR₅ et ensuite sur un composé de formule Cl-CH₂-COOR₄, R₄ et R₅ possèdent les significations donnée à la formule (3a).
